# EUROPEAN PATENT APPLICATION

(11) **EP 2 219 026 A1**
(43) Date of publication of application: **18.08.2010**
(21) Application number: 09001973.8
(22) Date of filing: 12.02.2009
(51) Int. Cl.: G01N 33/20, G01N 1/28

(54) **Method to determine the quality of a component**

(71) Applicant: Siemens Aktiengesellschaft, 80333 München (DE)
(72) Inventor: Poulsen, Knud Erik, 8270 Hojbjerg (DK)

(57) **Abstract**

The invention relates to a method and to an arrangement to determine the quality of a metal-component.
A predetermined area of the component-surface is prepared for a surface-based metallurgical testing-method to discover contaminations of the area. A replica is taken from the predetermined area, so the replica shows number and size of the discovered contaminations. The replica is analysed to detect the quality of the component based on the contaminations, which are shown by the replica.

## Description

The invention relates to a method to determine the quality of a metal-made component.

The life-time of a wind-turbine or any other machine is mainly predetermined by the quality of its components. The quality of metal-components, especially made of steel like like bearings, gears, etc., determines the live-time of a wind-turbine.

To achieve a good steel-quality a big amount of fluid steel is produced into a bottle-shaped bulb. The steel is cooled down and is brought into a specific form. Certain contaminations, which are caused by the steel-producing-process, gather at a known volume of this form. The contaminations are typically non-metallic and are mainly caused by the heat-resistance-surface of the bulb. The volume containing the contaminations is cut away, while high-quality steel remains for further processing.

There is a growing need for steel all over the world, especially in China, leading to a decrease of steel on the world-markets. Because of this the steel-producing-industry increases their steel-output by cutting off smaller amounts of the contaminated volume. So the steel-quality decreases step by step as more contaminations remain inside the steel, before it is further processed.

It is possible to examine the quality of metal before it is used to build needed components. This is necessary to reduce failures based on a poor metal-quality.

For this a so called "destructive" metallurgical testing-method is used. A piece of the metal is cut out and is brought to a laboratory. There its surface is grinded, polished and acid-corroded for an analyse by help of a microscope.

With this it is possible to detect cracks and even inclusions, caused by contaminations of the metal. Cracks may lead, if disregarded, to huge damages inside the wind-turbine, where metal-made-components like bearings or gears have to withstand high fatigue-loads.

The inclusions influence the number of revolutions and therefore the life-time of the used bearings. For example an inclusion-size between 30 micrometer and 60 micrometer lead to a life-time of 15 million up to 75 million revolutions of a bearing.

Very clean but expensive high-quality-steel show inclusions, which are smaller than 30 micrometer. With this a bearing-life-time of greater than 200 million revolutions can be achieved.

Inclusions with a size of 90 micrometer lead to a life-time of only 3 million revolutions of the bearing, for example.

The destructive-testing-method is the known method to determine the quality of metal-made components. But it can be only used without great disadvantageous, as it destroys the component or as it can be only used before the component itself is produced or mounted inside a machine or a wind-turbine.

It is therefore the aim of the invention, to provide an improved method and a matched arrangement for this method to determine the quality of metal-components.

This aim is solved by the features of claim 1 and of claim 15. Preferred embodiments are subject of the dependent claims.

According to the invention the quality of a metal-component is determined. A predetermined area of the component-surface is prepared for a surface-based metallurgical testing-method to discover contaminations of the area. A replica is taken from the predetermined area, so the replica shows number and size of the discovered contaminations. The replica is analysed to detect or to determine the quality of the component based on the contaminations, which are shown by the replica.

The invention is based on the fact, that normally contaminations of the component are spread inside the metal, so it is possible to determine its quality by an evaluation of a predetermined part of the surface.

The inventive method is a non-destructive testing-method, so it can be applied even to components, which are still mounted and/or which are still at work inside a machine or wind-turbine.

The inventive method is even applicable to detect cracks, which may appear, if the component is exposed to a fatigue-load at its work.

Based on the invention it is also possible to determine a threshold limit for a stress intensity factor, which initiates cracks and their growth.

Based on a number of taken replicas it is also possible to establish a data-base, which can be used for statistical purposes and predictions also in view of the life-time of tested components.

It is also possible to combine the inventive method with known destructive testing methods to improve the data-base.

By this the influence of bigger or smaller defects in relation to the life-time of known components can be determined.

Based on the gathered data it is possible to determine a maximum defect-size of inclusions in a material by help of the "Statistics of Extreme Values, SEV"-method, for example the ASTM E2283-03 "Standard Practice for Extreme Value Analysis of Nonmetallic Inclusions in Steel and other Microstructural Features".

The inventive method even allow an incoming inspection of stressed components, which were already at use.

The inventive method allows the testing of components at every point of time, whenever this is needed.

The inventive method allows also to improve and to verify life-time predictions.

According to the invention it is also possible to monitor an outer ring and/or an inner ring of bearings, rollers of the bearings and of flanges of the bearings or other components of a wind-turbine.

The invention will be shown in more detail by help of the following drawings.
- FIG 1: shows a microscopic picture of a surface of a main-bearing, which is analysed as component,
- FIG 2: shows a microscopic picture of the surface of a replica taken from the component of FIG 1, and
- FIG 3: shows the inventive use of the replica according to the invention.

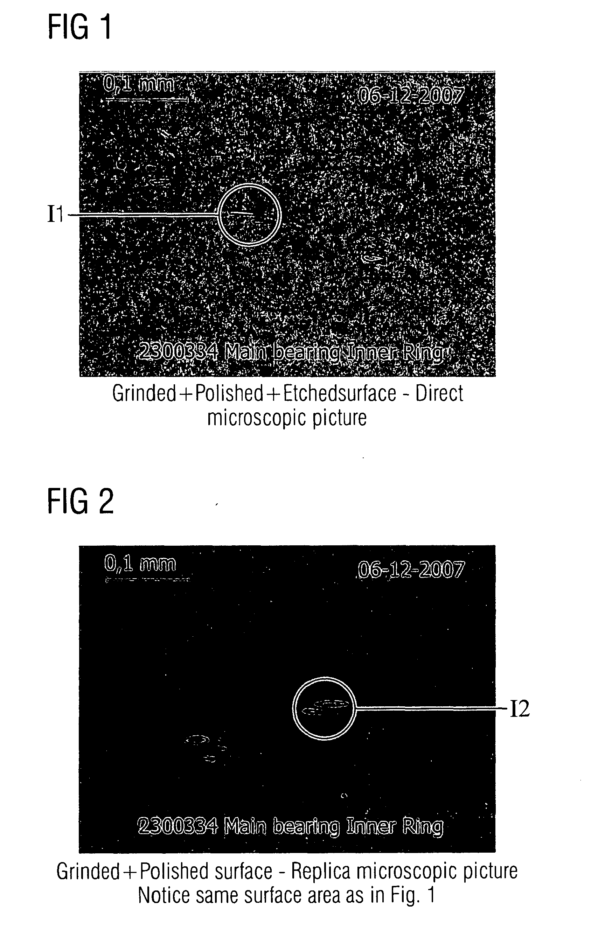

FIG 1 shows a microscopic picture of a surface of a steel-made main-bearing, to be analysed as component. The surface was grinded, polished, acid-corroded and cut out to determine a number and the size of inclusions I1. Even cracks might be detected - not shown in FIG 1 in detail.

FIG 2 shows a microscopic picture of the surface of a replica, taken from the component of FIG 1.

The replica shows for example inclusions I2. It can be seen that the size of the inclusions I1 and I2 are consistent with each other, so it is possible to determine the quality of the mounted main-bearing based on the replica.

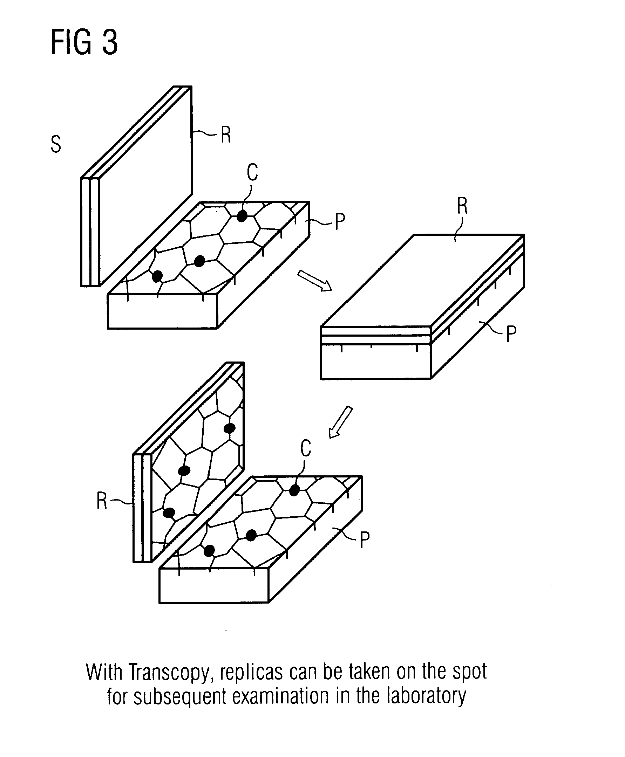

FIG 3 shows the inventive use of the replica R according to the invention.

A component P, made of steel for example, of a machine needs to be analyzed.

A replica R is taken from a predetermined area of the component P and is brought to a laboratory for further proceedings.

In a preferred embodiment a non-functional area, for example a non-stressed area of a main-bearing, is used to analyse the quality of the component.

The replica R shows the size and the number of inclusions and/or cracks C, which are assigned to the area of the component P.

The replica R is analysed beyond, while the component P remains inside the machine. So the inventive method is easy to be used without a long-lasting interruption of the machine, where the component is mounted into.

The quality and even the life-time of the component can be determined. In preferred embodiment a statistical data-base is used for this purpose.

## Claims

1. Method to determine the quality of a component,
- where the component is made of metal,
- where a predetermined area of the component-surface is prepared for a surface-based metallurgical testing-method to discover contaminations of the area,
- where a replica is taken from the predetermined area, so the replica shows number and size of the discovered contaminations,
- where the replica is analysed to detect the quality of the component based on the contaminations, which are shown by the replica.

2. Method according to claim 1, where the component is made of steel.

3. Method according to claim 1, where the contaminations are caused by inclusions, which are dependant to the production process of the metal.

4. Method according to claim 1, where the predetermined area is grinded, polished and/or corroded to detect contaminations, tracks of contaminations and/or cracks, which are allocated to the area.

5. Method according to claim 1, where a part of a non-functional surface of the component is used as predetermined area.

6. Method according to claim 5, where the used non-functional surface of the component is not exposed to fatigue-loads.

7. Method according to claim 1, where the component is part of a bearing or part of a gear.

8. Method according to claim 1, where the replica is taken from a component, which is already mounted inside a wind-turbine or a machine.

9. Method according to claim 1, where the life-time of the component is calculated based on the metal-contaminations.

10. Method according to claim 1, where a number of predetermined areas is used to take a number of allocated replicas.

11. Method according to claim 10, where the detected contaminations of a number of components are used to build a database, which is used to rate the metal-quality and/or to predict the live-time of other tested components based on a statistical method.

12. Method according to claim 11, where the statistical method uses size and number of cracks and/or inclusions to predict the live-time or to rate the quality.

13. Method according to claim 11 or 12, where the statistical method is used to predict a maximum size of an inclusion, which is located inside the component.

14. Method according to claim 1, where a casting-material with a low viscosity and/or a quick-hardening-casting-material is used to form the replica.

15. Arrangement to determine the quality of a component with devices, being built to perform the method-steps according to claim 1 to 14.
